# EUROPEAN PATENT APPLICATION

(11) **EP 2 248 523 A1**
(43) Date of publication of application: **10.11.2010**
(21) Application number: 09159537.1
(22) Date of filing: 06.05.2009
(51) Int. Cl.: A61K 31/519, A61K 31/616, A61K 45/06, A61P 9/00

(54) **Compounds for use in the treatment of clinical conditions resulting from a deficit of endothelial progenitor cells**

(71) Applicant: Universität zu Köln, 50923 Köln (DE)
(72) Inventor: Taubert, Dirk, 51429, Bergisch Gladbach (DE); Jung, Norma, 50931, Köln (DE); Koch, Nora, 50354, Hürth (DE)
(74) Representative: Althausen, Sonja

(57) **Abstract**

The present invention relates to the use of adenosine level affecting-compounds selected from the group comprising dipyridamole, mopidamol, dilazep, draflazine, 2,6-Bis(diethanolamino)-4,8-diheptamethyleneimino-pyrimido[5,4-d]pyrimidine, RA-642, adenosine, adenosine analogues, adenosine A_{2(A)} receptor agonists, inhibitors of adenosine kinase, activators of ecto-5'-nucleotidase and/or pharmaceutically acceptable salts thereof for the manufacture of a medicament for the therapeutic or prophylactic treatment and/or diagnosis of clinical conditions resulting from a deficit of endothelial progenitor cells.

## Description

The present invention relates to the treatment of clinical conditions resulting from a deficit of endothelial progenitor cells, using adenosine level affecting-compounds such as dipyridamole, adenosine, or adenosine analogues.

Endothelial progenitor cells are bone-marrow-derived cells that circulate in the blood and have the ability to differentiate into endothelial cells. The amount of endothelial progenitor cells is affected by numerous anthropometrical and clinical factors such as age, gender, body mass index, physical activity, and medicaments. A decreased number and function of endothelial progenitor cells was observed in patients suffering from different disorders. However, the reason for decreased levels of endothelial progenitor cells and the clinical effect still remains unclear. Experimental therapy of a deficit of endothelial progenitor cells currently is limited to autogenic or allogenic infusion of endothelial progenitor cells or stem cells. Such infusions are complex and the therapeutic success at present is limited.

Therefore, new therapeutic modalities for the treatment of clinical conditions resulting from a deficit of endothelial progenitor cells are urgently needed.

Dipyridamole also denoted N,N'-(4,8-Dipiperidinopyrimido[5,4-d]pyrimidine or 2,2',2",2"'-(4,8-di(piperidin-1-yl)pyrimido[5,4-d]pyrimidine-2,6-diyl)bis(azanetriyl)tetraethanol according to the IUPAC nomenclature is a drug that is for example used together with acetyl salicylic acid in the secondary prevention of stroke and transient ischaemic attack.

The object underlying the present invention was to provide a novel agent for the treatment or diagnosis of clinical conditions resulting from a deficit of endothelial progenitor cells.

This object is met with means according to the independent claims of the present invention. The dependent claims are related to preferred embodiments.

The term "adenosine level affecting-compounds" as used herein refers to or describes compounds or substances which affect, preferably increase, the level of adenosine in the plasma. The plasma adenosine level can be increased by administering adenosine analogues or by using drugs such as dipyridamole which affect the transport, synthesis or degradation of adenosine, for example by decreasing the activity of adenosine kinase or by activating the activity of the ecto-5'-nucleotidase.

As used herein, the term "adenosine-depending deficit of endothelial progenitor cells" refers to a deficit of endothelial progenitor cells (EPC) that depends on the adenosine level. Surprisingly it was found that a deficit of adenosine in the plasma can result in a deficit of endothelial progenitor cells.

The term "clinical condition" as used herein refers to a condition in a human or animal that impairs the health and well being of the human or animal and which may take the form of well documented disease states such as coronary heart disease or a general condition of poor health brought about by a deficit of endothelial progenitor cells.

The term adenosine "analogue" as used herein refers to a chemical compound with a preferably slightly altered chemical structure compared to adenosine.

As used herein, the term "prophylactic treatment" refers to either preventing or inhibiting the development of a clinical condition or disorder or delaying the onset of a pre-clinically evident stage of a clinical condition or disorder.

The term "therapeutically effective amount" is used herein to mean an amount or dose sufficient to cause an improvement in a clinically significant condition in the subject.

The term "biological sample", as used herein, refers to a sample obtained from a patient. The sample may be of any biological tissue or fluid. Such samples preferably include blood, serum, plasma, or blood cells (e.g., white cells).

Unless otherwise defined, the technical and scientific terms used herein have the same meaning as commonly understood by one of ordinary skill in the art to which this invention belongs.

The present invention is directed to the use of adenosine level affecting-compounds selected from the group comprising dipyridamole, mopidamol, dilazep, draflazine, 2,6-Bis(diethanolamino)-4,8-diheptamethyleneimino-pyrimido[5,4-d]pyrimidine, RA-642, adenosine, adenosine analogues, adenosine A_{2(A)} receptor agonists, inhibitors of adenosine kinase, activators of ecto-5'-nucleotidase and/or pharmaceutically acceptable salts thereof for the manufacture of a medicament for the therapeutic or prophylactic treatment and/or diagnosis of clinical conditions resulting from a deficit of endothelial progenitor cells.

Furthermore, the invention relates to a pharmaceutical or diagnostic composition for the therapeutic and/or prophylactic treatment and/or the diagnosis of clinical conditions resulting from a deficit of endothelial progenitor cells. Preferred embodiments are given in the dependant claims.

Surprisingly, it was found that the adenosine level especially in the blood plasma can affect the level of endothelial progenitor cells. Beneficially, further it was found that the use of adenosine level affecting-compounds, preferably compounds that affect an increase in the adenosine level such as dipyridamole can exhibit effects in the treatment of clinical conditions resulting from a deficit of endothelial progenitor cells such as coronary heart disease.

Further surprisingly, it was discovered that adenosine level affecting-compounds such as dipyridamole can increase the viability of endothelial progenitor cells. It is particularly advantageous that a use of adenosine level affecting-compounds such as dipyridamole can induce an increase of the level of endothelial progenitor cells and can reduce the amount of apoptotic endothelial progenitor cells.

Therefore, preferred compounds are adenosine level increasing-compounds. Further, a preferred deficit of endothelial progenitor cells is an adenosine-depending deficit of endothelial progenitor cells.

Dipyridamole also is denoted N,N'-(4,8-Dipiperidinopyrimido[5,4-d]pyrimidine or 2,2',2",2"'-(4,8-di(piperidin-1-yl)pyrimido[5,4-d]pyrimidine-2,6-diyl)bis(azanetriyl)tetraethanol according to the IUPAC nomenclature. Mopidamol also is denoted 2,2',2,2'-((4-(1-piperidinyl)pyrimido(5,4-d)pyrimidine- 2,6-diyl)dinitrilo)tetrakisethanol. Dilazep according to the IUPAC nomenclature is denoted 3-(4-{3-[(3,4,5-trimethoxyphenyl)carbonyloxy]propyl}-1,4-diazepan-1-yl)propyl 3,4,5-trimethoxybenzoate. Draflazine also is denoted 2-aminocarbonyl-N-(4-amino-2,6-dichlorophenyl)-4-(5,5-bis(4-fluorophenyl)-pentyl)-1-piperazineacetamide. RA-642 also is denoted (2,2'-(4,8-bis(diethylamino)pyrimido(5,4-d)pyrimidine-2,6-diyl)di(2-methoxyethyl)imino)diethanol.

Preferably, the adenosine level affecting-compounds such as dipyridamole can be used in form of pharmaceutically acceptable salts thereof, preferably as acid addition salts. Preferred acid addition salts usually are hydrochloride salts or hydrochloride hydrates.

A preferred deficit of endothelial progenitor cells is an adenosine-depending deficit of endothelial progenitor cells. A preferred adenosine level affecting-compounds is dipyridamo le.

The present invention focuses on the effect of adenosine affecting the level of endothelial progenitor cells, especially an adenosine-depending deficit of endothelial progenitor cells. Various conditions and disorders can be caused by a deficit of endothelial progenitor cells, which may result in disease states such as coronary heart disease.

The present invention therefore provides a novel approach for the treatment of clinical conditions resulting from a deficit of endothelial progenitor cells.

Preferred clinical conditions resulting from a deficit of endothelial progenitor cells, particularly an adenosine-depending deficit of endothelial progenitor cells, are diseases selected from the group comprising coronary heart disease and/or vascular diseases. Vascular diseases preferably are selected from the group comprising atherosclerosis, diabetes mellitus, hypertension, metabolic syndrome, ischemic stroke, peripheral arterial disease, valvular heart disease, Raynaud syndrome, erectile dysfunction, chronic obstructive pulmonary disease (COPD), rheumatoid arthritis and other autoimmune disorders with vascular involvement, human immunodeficiency virus (HIV) infection and other chronic infective disorders with vascular involvement, retinal vascular defects, graft vasculopathy, renal insufficiency and/or hemodialysis.

A preferred disorder or indication according to the present invention is coronary heart disease.

The adenosine level affecting-compounds can be administered orally, intravenously, topically, or via other standard routes. Dipyridamole formulations may be in the form of solutions or suspensions for injection, or be in capsule, tablet, dragee, or other solid composition or as a solution or suspension for oral administration. Preferably, dipyridamole can be administered using any of the oral dipyridamole retard, instant or parenteral formulations on the market, wherein the retard formulations are preferred. In preferred embodiments, dipyridamole is formulated as a sustained release formulation, preferably as an oral sustained release formulation. Preferably, the sustained release formulation is selected from the group comprising sustained release capsules and/or sustained release tablets. Sustained release capsules preferably are selected from the group comprising hard gelatine retard capsules, and soft gelatine retard capsules.

Advantages from dipyridamole release in sustained release formulations are a more uniform plasma drug profile and a smoother therapeutic response over the dosage interval.

The adenosine level affecting-compounds such as dipyridamole can be administered as a single medication or as a combination with one or more further compounds.

In preferred embodiments dipyridamole is used in combination with a compound selected from the group comprising acetylsalicylic acid, statins, ACE (Angiotensin-Converting Enzyme) inhibitors, PPARgamma (Peroxisome Proliferator Activated Receptor gamma) agonists, glucocorticoids, clopidogrel, ticlopidin, prasugrel, ticagrelor, cangrelor, PRT 060128 and/or SCH 530348.

Clopidogrel is denoted according to the IUPAC nomenclature (+)-(S)-methyl 2-(2-chlorophenyl)-2-(6,7-dihydrothieno[3,2-c]pyridin-5(4H)-yl)acetate (clopidogrel). Ticlopidine is denoted according to the IUPAC nomenclature 5-(2-chlorobenzyl)-4,5,6,7-tetrahydrothieno[3,2-c]pyridine. Prasugrel is denoted according to the IUPAC nomenclature 5-[2-cyclopropyl-1-(2-fluorophenyl)-2-oxoethyl]-4,5,6,7-tetrahydrothieno[3,2-c]pyridin-2-yl acetate. Ticagrelor is denoted according to the IUPAC nomenclature (1S,2S,3R,5S)-3-[7-[(1R,2S)-2-(3,4-Difluorophenyl)cyclopropylamino]-5-(propylthio)-3H-[1,2,3]triazolo[4,5-d]pyrimidin-3-yl]-5-(2-hydroxyethoxy)cyclopentane-1,2-diol. PRT- 060128 is also denoted elinogrel. SCH 530348 is also denoted Ethyl N-[(3R,3aS,4S,4aR,7R,8aR,9aR)-4-[(E)-2-[5-(3-fluorophenyl)-2-pyridyl]vinyl]-3-methyl-1-oxo-3a,4,4a,5,6,7,8,8a,9,9a-decahydro-3H-benzo[f]isobenzofuran-7-yl]carbamate.

The most useful compound to be used in combination with dipyridamole is acetyl salicylic acid. Dipyridamole sustained release formulations in combination with low-dose acetyl salicylic acid is available on the market, available for example under the trademarks Aggrenox® or Asasantin®.

Useful statins for use in combination with dipyridamole are preferably selected from the group comprising simvastatin, pravastatin, fluvastatin, lovastatin, atorvastatin and/or rosuvastatin. A useful ACE inhibitor for use in combination with dipyridamole is for example Lisinopril. Useful PPARgamma (Peroxisome Proliferator Activated Receptor gamma) agonists for use in combination with dipyridamole are preferably selected from the group comprising rosiglitazone and/or pioglitazone. Useful glucocorticoids for use in combination with dipyridamole are preferably selected from the group comprising prednisone and/or prednisolone.

In preferred embodiments, dipyridamole can be administered orally in a daily dosage in the range of ≥ 25 mg to ≤ 500 mg, preferably in the range of ≥ 100 mg to ≤ 400 mg, more preferably in the range of ≥ 300 mg to ≤ 400 mg.

For long-term treatment it can be advantageously to administer repeated doses of dipyridamole several times a day, for example a dosage in the range of ≥ 200 mg to ≤ 250 mg dipyridamole two times daily, or a dosage in the range of ≥ 75 mg to ≤ 100 mg dipyridamole four times daily. Preferably, dipyridamole is administered as a sustained release formulation several times a day.

Advantageously, it was found that adenosine, adenosine analogues, adenosine A_{2(A)} receptor agonists, inhibitors of adenosine kinase, and/or activators of ecto-5'-nucleotidase also can increase the level of endothelial progenitor cells.

In preferred embodiments, the adenosine analogues are selected from the group comprising 5'-N-Ethylcarboxamidoadenosine (NECA), L-phenylisopropyl-adenosine (L-PIA), 2-chloroadenosine and/or N6-cyclopentyladenosine.

In further preferred embodiments, the adenosine A_{2(A)} agonists are selected from the group comprising 2-p-(2-Carboxyethyl)phenethylamino-5'-N-ethylcarboxamidoadenosine.

A preferred acid addition salt of2-p-(2-Carboxyethyl)phenethylamino-5'-N-ethylcarboxamidoadenosine is 2-p-(2-Carboxyethyl)phenethylamino-5'-N-ethylcarboxamidoadenosine hydrochloride hydrate (CGS-21680).

A preferred inhibitor of adenosine kinase is 5'-Iodotubericidine. Preferred activators of ecto-5'-nucleotidase are selected from the group comprising bleomycin and/or dexamethasone.

Preferably, adenosine and adenosine analogues are administered orally.

The present invention also relates to adenosine level affecting-compounds selected from the group comprising dipyridamole, mopidamol, dilazep, draflazine, 2,6-Bis(diethanolamino)-4,8-diheptamethyleneimino-pyrimido[5,4-*d*]pyrimidine, RA-642, adenosine, adenosine analogues, adenosine A_{2(A)} receptor agonists, inhibitors of adenosine kinase, activators of ecto-5'-nucleotidase and/or pharmaceutically acceptable salts thereof for use in the therapeutic or prophylactic treatment and/or diagnosis of clinical conditions resulting from a deficit of endothelial progenitor cells.

A preferred deficit of endothelial progenitor cells is an adenosine-depending deficit of endothelial progenitor cells. A preferred adenosine level affecting-compounds is dipyridamo le.

Whereas the activity of dipyridamole as a platelet aggregation inhibitor is well known it is a new finding that the compounds usable according to the invention additionally are usable in the treatment of clinical conditions resulting from a deficit of endothelial progenitor cells.

Preferably, the adenosine level affecting-compounds such as dipyridamole can be used as pharmaceutically acceptable salts thereof, preferably as acid addition salts. Preferred acid addition salts usually are hydrochloride salts or hydrochloride hydrates.

Preferred clinical conditions resulting from a deficit of endothelial progenitor cells particularly an adenosine-depending deficit of endothelial progenitor cells are diseases selected from the group comprising coronary heart disease and/or vascular diseases. Vascular diseases preferably are selected from the group comprising atherosclerosis, diabetes mellitus, hypertension, metabolic syndrome, ischemic stroke, peripheral arterial disease, valvular heart disease, Raynaud syndrome, erectile dysfunction, chronic obstructive pulmonary disease (COPD), rheumatoid arthritis and other autoimmune disorders with vascular involvement, human immunodeficiency virus (HIV) infection and other chronic infective disorders with vascular involvement, retinal vascular defects, graft vasculopathy, renal insufficiency and/or hemodialysis. A most preferred disorder or indication to be treated with the use according to the present invention is coronary heart disease.

The adenosine level affecting-compounds can be administered orally, intravenously, topically, or via other standard routes. Dipyridamole formulations may be in the form of solutions or suspensions for injection, or be in capsule, tablet, dragee, or other solid composition or as a solution or suspension for oral administration. Preferably, dipyridamole can be administered using any of the oral dipyridamole retard, instant or parenteral formulations on the market, wherein the retard formulations are preferred. In preferred embodiments, dipyridamole is formulated as a sustained release formulation, preferably as an oral sustained release formulation. Preferably, the sustained release formulation is selected from the group comprising sustained release capsules and/or sustained release tablets. Sustained release capsules preferably are selected from the group comprising hard gelatine retard capsules, and soft gelatine retard capsules.

The adenosine level affecting-compounds such as dipyridamole can be administered as a single medication or as a combination with one or more further compounds. In preferred embodiments dipyridamole is used in combination with a compound selected from the group comprising acetylsalicylic acid, statins, ACE inhibitors, PPARgamma agonists, glucocorticoids, clopidogrel, ticlopidin, prasugrel, ticagrelor, cangrelor, PRT 060128 and/or SCH 530348.

A most useful compound to be used in combination with dipyridamole is acetyl salicylic acid. Dipyridamole sustained release formulations in combination with low-dose acetyl salicylic acid is available on the market, available for example under the trademarks Aggrenox® or Asasantin®.

Useful statins for use in combination with dipyridamole are preferably selected from the group comprising simvastatin, pravastatin, fluvastatin, lovastatin, atorvastatin and/or rosuvastatin. A useful ACE inhibitor for use in combination with dipyridamole is for example lisinopril. Useful PPARgamma agonists for use in combination with dipyridamole are preferably selected from the group comprising rosiglitazone and/or pioglitazone. Useful glucocorticoids for use in combination with dipyridamole are preferably selected from the group comprising prednisone and/or prednisolone.

In preferred embodiments, dipyridamole can be administered orally in a daily dosage in the range of ≥ 25 mg to ≤ 500 mg, preferably in the range of ≥ 100 mg to ≤ 400 mg, more preferably in the range of ≥ 300 mg to ≤ 400 mg.

For long-term treatment it can be advantageously to administer repeated doses of dipyridamole several times a day, for example a dosage in the range of ≥ 200 mg to ≤ 250 mg dipyridamole two times daily, or a dosage in the range of ≥ 75 mg to ≤ 100 mg dipyridamole four times daily. Preferably, dipyridamole is administered as a sustained release formulation several times a day.

In preferred embodiments, the adenosine analogues are selected from the group comprising 5'-N-Ethylcarboxamidoadenosine (NECA), L-phenylisopropyl-adenosine (L-PIA), 2-chloroadenosine and/or N6-cyclopentyladenosine.

In further preferred embodiments, the adenosine A_{2(A)} agonists are selected from the group comprising 2-p-(2-Carboxyethyl)phenethylamino-5'-N-ethylcarboxamidoadenosine.

A preferred acid addition salt of2-p-(2-Carboxyethyl)phenethylamino-5'-N-ethylcarboxamidoadenosine is 2-p-(2-Carboxyethyl)phenethylamino-5'-N-ethylcarboxamidoadenosine hydrochloride hydrate (CGS-21680).

A preferred inhibitor of adenosine kinase is 5'-Iodotubericidine. Preferred activators of ecto-5'-nucleotidase are selected from the group comprising bleomycin and/or dexamethasone.

Another aspect according to the present invention is a pharmaceutical or diagnostic composition for the therapeutic and/or prophylactic treatment and/or the diagnosis of clinical conditions resulting from a deficit of endothelial progenitor cells, particularly an adenosine-depending deficit of endothelial progenitor cells, comprising an adenosine level affecting-compound selected from the group comprising dipyridamole, mopidamol, dilazep, draflazine, 2,6-Bis(diethanolamino)-4,8-diheptamethyleneimino-pyrimido[5,4-*d*]pyrimidine, RA-642, adenosine, adenosine analogues, adenosine A_{2(A)} receptor agonists, inhibitors of adenosine kinase, activators of ecto-5'-nucleotidase and/or pharmaceutically acceptable salts thereof.

The present invention also relates to methods for detecting and/or diagnosing the presence of a clinical condition resulting from a deficit of endothelial progenitor cells, particularly an adenosine-depending deficit of endothelial progenitor cells, in a subject, comprising detecting and/or quantifying the level of adenosine in the plasma and/or erythrocytes in a biological sample obtained from said subject.

The method may involve detecting and/or quantifying adenosine-depending activity markers such as adenosine kinase, methylthioadenosine phosphorylase (MTAP), adenosine deaminase (ADA), CD39, CD73, equilibrative and concentrative nucleoside transporters (ENT1-4, CNT1-3), hypoxia-inducible factor 1 (HIF-1) and/or vascular endothelial growth factor (VEGF), preferably in peripheral blood mononuclear cells (PBMC).

The means of detection and/or quantification may involve quantifying the expression of activity markers such as adenosine kinase by real-time PCR or flow cytometry methods. The means of detection and/or quantification further may involve quantifying methylthioadenosine phosphorylase expression by real-time PCR methods, determining adenosine kinase activity by quantifying the rate of conversion of etheno adenosine to etheno adenosine mono phosphate (E-AMP) or CD73 activity by quantifying the rate of conversion of E-AMP to etheno adenosine by liquid chromatography-mass spectrometry such as LC-MS/MS.

Further, the invention relates to a method of detecting and/or diagnosing a clinical condition resulting from a deficit of endothelial progenitor cells in a subject, the method comprising the steps of:
- providing a biological sample from a patient;
- detecting and/or quantifying the level of adenosine in the plasma and/or erythrocytes of the biological sample;
- detecting and/or quantifying the level of adenosine-depending activity markers;
- determining the standardised number of endothelial progenitor cells;
- determining the correlation of adenosine and adenosine-depending activity markers with the standardised number of endothelial progenitor cells; and
- comparing the level of adenosine in the plasma and/or erythrocytes of the biological sample with that in a control sample,
wherein a different expression level of adenosine and/or adenosine-depending activity markers in the biological sample compared to that in the control sample indicates the presence of a clinical condition resulting from a deficit of endothelial progenitor cells in the patient.

Also encompassed within the scope of the invention is a method of treating a clinical condition resulting from a deficit of endothelial progenitor cells, particularly an adenosine-depending deficit of endothelial progenitor cells in a subject, the method comprising administering to the subject a therapeutically effective amount of an adenosine level affecting-compounds selected from the group comprising dipyridamole, mopidamol, dilazep, draflazine, 2,6-Bis(diethanolamino)-4,8-diheptamethyleneimino-pyrimido[5,4-*d*]pyrimidine, RA-642, adenosine, adenosine analogues, adenosine A_{2(A)} receptor agonists, inhibitors of adenosine kinase, activators of ecto-5'-nucleotidase and/or pharmaceutically acceptable salts thereof.

A preferred deficit of endothelial progenitor cells is an adenosine-depending deficit of endothelial progenitor cells.

A preferred adenosine level affecting-compounds is dipyridamole. Whereas the activity of dipyridamole as a platelet aggregation inhibitor is well known it is a new finding that the compounds usable according to the invention additionally are usable in the treatment of clinical conditions resulting from a deficit of endothelial progenitor cells.

Preferably, the adenosine level affecting-compounds such as dipyridamole can be used as pharmaceutically acceptable salts thereof, preferably as acid addition salts. Preferred acid addition salts usually are hydrochloride salts or hydrochloride hydrates.

Preferred clinical conditions resulting from a deficit of endothelial progenitor cells particularly an adenosine-depending deficit of endothelial progenitor cells are diseases selected from the group comprising coronary heart disease and/or vascular diseases. Vascular diseases preferably are selected from the group comprising atherosclerosis, diabetes mellitus, hypertension, metabolic syndrome, ischemic stroke, peripheral arterial disease, valvular heart disease, Raynaud syndrome, erectile dysfunction, chronic obstructive pulmonary disease (COPD), rheumatoid arthritis and other autoimmune disorders with vascular involvement, human immunodeficiency virus (HIV) infection and other chronic infective disorders with vascular involvement, retinal vascular defects, graft vasculopathy, renal insufficiency and/or hemodialysis. A most preferred disorder or indication to be treated with the use according to the present invention is coronary heart disease.

The adenosine level affecting-compounds can be administered orally, intravenously, topically, or via other standard routes. Dipyridamole formulations may be in the form of solutions or suspensions for injection, or be in capsule, tablet, dragee, or other solid composition or as a solution or suspension for oral administration. Preferably, dipyridamole can be administered using any of the oral dipyridamole retard, instant or parenteral formulations on the market, wherein the retard formulations are preferred. In preferred embodiments dipyridamole is formulated as a sustained release formulation, preferably as an oral sustained release formulation. Preferably, the sustained release formulation is selected from the group comprising sustained release capsules and/or sustained release tablets. Sustained release capsules preferably are selected from the group comprising hard gelatine retard capsules, and soft gelatine retard capsules.

The adenosine level affecting-compounds such as dipyridamole can be administered as a single medication or as a combination with one or more further compounds. In preferred embodiments dipyridamole is used in combination with a compound selected from the group comprising acetylsalicylic acid, statins, ACE inhibitors, PPARgamma agonists, glucocorticoids, clopidogrel, ticlopidin, prasugrel, ticagrelor, cangrelor, PRT 060128 and/or SCH 530348.

A most useful compound to be used in combination with dipyridamole is acetyl salicylic acid. Dipyridamole sustained release formulations in combination with low-dose acetyl salicylic acid is available on the market, available for example under the trademarks Aggrenox® or Asasantin®.

Useful statins for use in combination with dipyridamole are preferably selected from the group comprising simvastatin, pravastatin, fluvastatin, lovastatin, atorvastatin and/or rosuvastatin. A useful ACE inhibitor for use in combination with dipyridamole is for example lisinopril. Useful PPARgamma agonists for use in combination with dipyridamole are preferably selected from the group comprising rosiglitazone and/or pioglitazone. Useful glucocorticoids for use in combination with dipyridamole are preferably selected from the group comprising prednisone and/or prednisolone.

In preferred embodiments, dipyridamole can be administered orally in a daily dosage in the range of ≥ 25 mg to ≤ 500 mg, preferably in the range of ≥ 100 mg to ≤ 400 mg, more preferably in the range of ≥ 300 mg to ≤ 400 mg.

For long-term treatment it can be advantageously to administer repeated doses of dipyridamole several times a day, for example a dosage in the range of ≥ 200 mg to ≤ 250 mg dipyridamole two times daily, or a dosage in the range of ≥ 75 mg to ≤ 100 mg dipyridamole four times daily. Preferably, dipyridamole is administered as a sustained release formulation several times a day.

In preferred embodiments, the adenosine analogues are selected from the group comprising 5'-N-Ethylcarboxamidoadenosine (NECA), L-phenylisopropyl-adenosine (L-PIA), 2-chloroadenosine and/or N6-cyclopentyladenosine.

In further preferred embodiments, the adenosine A_{2(A)} agonists are selected from the group comprising 2-p-(2-Carboxyethyl)phenethylamino-5'-N-ethylcarboxamidoadenosine.

A preferred acid addition salt of2-p-(2-Carboxyethyl)phenethylamino-5'-N-ethylcarboxamidoadenosine is 2-p-(2-Carboxyethyl)phenethylamino-5'-N-ethylcarboxamidoadenosine hydrochloride hydrate (CGS-21680).

A preferred inhibitor of adenosine kinase is 5'-Iodotubericidine. Preferred activators of ecto-5'-nucleotidase are selected from the group comprising Bleomycin and/or Dexamethasone.

Further features of the present invention will become apparent from the figures and examples, which, in an exemplary fashion, show preferred embodiments of the present invention. However, these examples should by no means be understood as to limit the scope of the invention.

Referring to the figures shows:

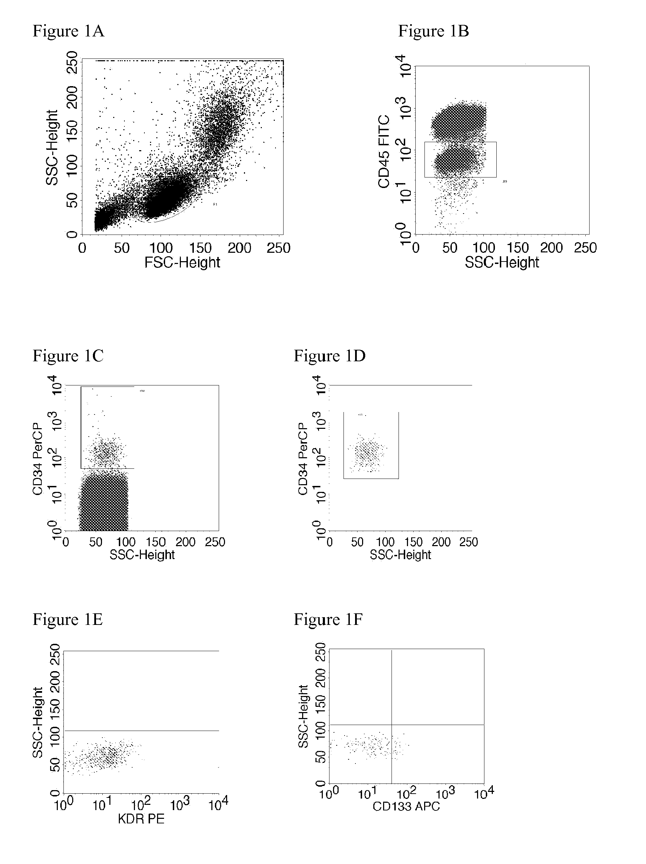

- Fig.1: Original recording of dot plots depicting the method of determination of EPC in PBMC by serial gating of different subpopulations of cells, wherein Figures 1A to 1F show respectively: (A) Forward-scatter / side-scatter plot of PBMC with lymphocyte gate, (B) CD45 staining of lymphocytes with CD45dim gate, (C) CD34 staining of lymphocytes with CD34+ gate, (D) Gating of CD34+ CD45dim cells, (E) KDR staining of CD34+ CD45dim cells showing the KDR+ fraction in the lower right quadrant, and (F) CD133 staining of KDR+ and CD34+ CD45dim cells showing the CD133+ fraction in the lower right quadrant.
- Fig.2: A Venn diagram depicting the principle of the EPC determination based on the expression of specific surface antigens on mononuclear blood cells.
- Fig.3: Endothelial progenitor cell count in peripheral blood after intake of dipyridamole and acetyl salicylic acid (A) or acetyl salicylic acid (B).
Values in Figures 3A and 3B represent means and SD. Overall significance was assessed by one-way repeated measures ANOVA. *P<.05, **P<.01, ***P<.001 indicate Holm-corrected significant differences of EPC levels compared with day 0.
- Fig.4: Endothelial progenitor cell count in peripheral blood after intake of adenosine. Values in Figure 4 represent means and SD. Overall significance was assessed by one-way repeated measures ANOVA. *P<.05, **P<.01, ***P<.001 indicate Holm-corrected significant differences of EPC levels compared with day 0 at 0h (pre-dose value).

### Example 1

### Study assessing the endothelial progenitor cell count after intake of dipyridamole and acetyl salicylic acid

A double-blinded, randomized, crossover trial assessing endothelial progenitor cell count determined by a validated flow cytometric technique in peripheral blood of six healthy volunteers after intake of dipyridamole and acetyl salicylic acid or acetyl salicylic acid alone was performed.

### Study Participants

Subjects were unpaid volunteers who responded on an invitation in participating in a 20-days trial. Eligible were healthy Caucasian volunteers of both sex between 20 and 45 years of age. Included subjects were students or non-manual workers, and had normal levels of plasma lipids and plasma glucose. Subjects were excluded if they had cardiovascular diseases, diabetes mellitus, hyper-/dyslipidemia, gastrointestinal diseases, hepatic and renal disorders, pulmonary diseases, coagulopathy, hematological disorders, cancer, psychiatric disorders, alcohol or drug dependence, seizure disorders, history of organ transplantation, surgery within the last 12 months, positive tests for HIV, hepatitis B or C, a body-mass index of more than 27.5 or less than 18.5 kg/m2, leisure physical activity of more than 6 MET per week, actively smoked tobacco within the last five years, were pregnant or lactating women, were regular users of medications, used any medication within the last two weeks before entry, were users of vitamin, mineral or bioactive food supplements. Participants were not allowed to use coffee or other caffeine containing foods during the whole study and during a 7-days run-in phase. 15 volunteers were screened by evaluation of medical history, physical examination, laboratory parameters and assessment of the subjects' habitual diet. 9 subjects did not meet eligibility criteria. The remaining 6 (3 women, 3 men, aged 25-42 years) were allocated to treatments with 2 capsules per day of Aggrenox® (extended release dipyridamole (200 mg) and acetyl salicylic acid (25 mg), Boehringer Ingelheim, Germany) or acetyl salicylic acid alone (25 mg acetyl salicylic acid, CT Arzneimittel, Germany). The study was performed in crossover fashion. In a randomized order all 6 subjects received both aggrenox and acetyl salicylic acid. After the initial phase with Aggrenox® or acetyl salicylic acid and a washout period of 7 days the subjects received the respective crossover treatment. All of them completed the study and were included in the per-protocol analysis.

### Study Design

Eligible subjects were sequentially allocated to Aggrenox® or acetyl salicylic acid alone in blocks of 1 woman and 1 man using a computer-generated random number sequence. To conceal allocation from investigators, instructed trained staff at a separate site not involved with the trial generated and maintained the randomization list and prepared the medications. To conceal allocation from participants capsules with Aggrenox® and capsules with acetyl salicylic acid alone of identical appearance and weight were provided in dated, sequentially numbered, sealed, non-transparent bags.
All clinical investigations, laboratory tests, data collection and data analysis were done by physicians and trained staff who were blinded to group assignment.
After a 7 day caffeine-free run-in and an overnight fast of 12 hours, subjects were allocated to receive in a crossover fashion over 3 days Aggrenox® every 12 hours or acetyl salicylic acid alone every 12 hours, followed by a washout of 7 days weeks. Plasma parameters under both interventions were assessed in each subject in the 12-hour fasting state between 8 and 10 a.m. on days 0 (pre-dose), 1, 2, 3, and 7 days after discontinuation of the treatment.
EPCs were measured before treatment (day 0), after 1, 2, and 3 days of treatment and 7 days after discontinuation of the treatment (post-dose).
Adverse events were monitored every day by interview (completing a 12-item checklist of symptoms including headache and nausea), physical examination, and laboratory testing. Adherence to the study protocol was confirmed after each dose by direct questioning and monitoring uptake of capsules. After intake of Aggrenox® 4 subjects reported headaches, two of them also nausea. After intake of acetyl salicylic acid one subject reported headaches and one subject upper gastrointestinal discomfort. Under both treatments no bleeding events were observed.

### Laboratory Analysis

Blood was drawn form the median cubital vein of the left arm into citrated CPT vacutainer tubes (BD Biosciences, San Jose, CA, USA), and peripheral blood mononuclear cells (PBMC) were isolated by immediate centrifugation according to the manufacturer's instruction. PBMC were washed twice in phosphate-buffered saline, counted, resuspended in 100 µl of a fluorescence-activated cell-sorting buffer consisting of phosphate-buffered saline, 2% (m/v) fetal calf serum (Sigma, Taufkirchen, Germany) and 0.05% (m/v) sodium azide (Sigma), each containing 10⁶ cells, and subjected to flow cytometry analysis. Endothelial progenitor cells (EPC) were analyzed in the PBMC fraction according to the modified validated ISHAGE (International Society of Hematotherapy and Graft Engineering) criteria by assessment of the surface expression of CD45, CD34, CD133, and KDR (VEGFR2) using a BD FACSCalibur with CellQuest Pro version 5.2.1 software. PBMC were incubated for 30 min at 4 °C with the fluorochrome conjugated mouse IgG₁ antihuman antibodies: CD45-FITC 5µl (BD), CD34-PerCP 5µl (BD), CD133-APC 5µl (Miltenyi Biotec, Bergisch Gladbach, Germany), KDR-PE 10 µl (R&D Systems, Minneapolis, MN, USA). Fluorochrome conjugated rat antimouse IgG₁ antibodies were used as isotype controls. 350,000 PBMC per tube were analyzed. Standardized EPC counts (per 1 Mio cells in the lymphocyte gate of total PBMC) were calculated by enumeration of isotype-corrected CD45dim/CD34+/KDR+ triple positive or the CD45dim/CD34+/KDR+/CD133+ quadruple positive lymphocytes (CD31+) in the PBMC fraction.

Figure 1 illustrates the validated and standardized flow cytometry method for a counting of circulating endothelial progenitor cells (EPC) in peripheral blood mononuclear cells (PBMC) and depicts the gating strategy and an original recording of the EPC enumeration.

Figures 1A to 1F show the original recording of dot plots depicting the method of determination of EPC in PBMC by serial gating of different subpopulations of cells, wherein the respective Figures show: (A) Forward-scatter / side-scatter plot of PBMC with lymphocyte gate. (B) CD45 staining of lymphocytes with CD45dim gate. (C) CD34 staining of lymphocytes with CD34+ gate. (D) Gating of CD34+ CD45dim cells (isotype control not shown). (E) KDR staining of CD34+ CD45dim cells showing the KDR+ fraction in the lower right quadrant (isotype control not shown). (F) CD133 staining of KDR+ and CD34+ CD45dim cells showing the CD133+ fraction in the lower right quadrant (isotype control not shown).

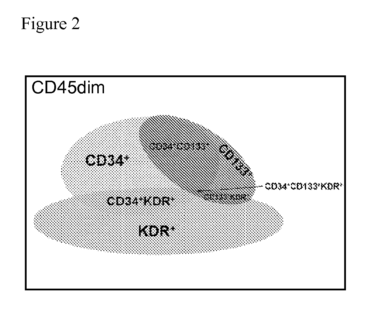

Figure 2 shows a Venn diagram depicting the principle of the EPC determination based on the expression of specific surface antigens on mononuclear blood cells. In the distinct subpopulation of cells showing intermediate expression of the common leukocyte antigen CD45 (CD45dim cells), EPC are determined by the co-expression of CD34 (CD34+), KDR (KDR+) and CD133 (CD133+). To limit variability and increase validity of the method (intraday and interday precision < 15%) the CD45dim/CD34+/KDR+ phenotype was considered more appropriate than the (higher specific) CD45dim/CD34+/KDR+/CD133+ phenotype if the total number of acquired events (PBMC) was below 700,000. To allow for comparability between EPC determinations absolute counts were standardized by calculating the numbers per 1 Mio of events in the lymphocyte gate.

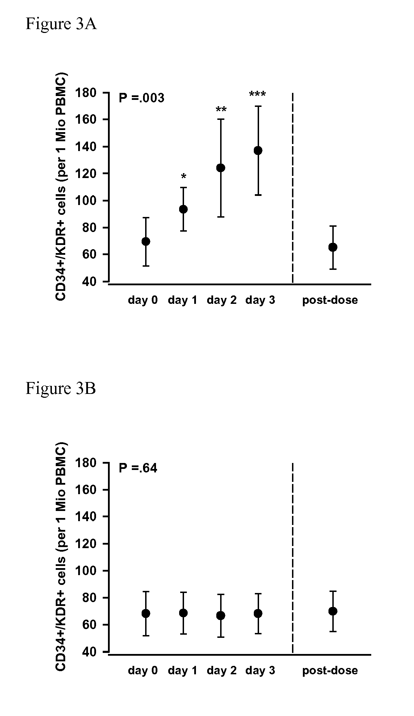

As can be seen from Figure 3A, the amount of endothelial progenitor cells increased already on day 1 and increased further on days 2 and 3 of the intake of dipyridamole and acetyl salicylic acid. After discontinuing the medication the amount of endothelial progenitor cells decreased to the pre-dose level.

As can be seen from Figure 3B, the amount of endothelial progenitor cells was not affected by intake of acetyl salicylic acid alone.

This indicates that dipyridamole alone was responsible for the increase in the number of progenitor cells. Dipyridamole acts as an inhibitor of ENT1, which is the crucial transporter involved in the uptake of circulating adenosine into blood cells and endothelial tissue. Hence, administration of dipyridamole elevates the plasma levels of adenosine and therefore, the plasma adenosine may be the causative factor regulating the EPC number.

### Example 2

Study assessing the endothelial progenitor cell count after intake of adenosine

The endothelial progenitor cell count was assed in a open-label trial in peripheral blood of 6 healthy volunteers (2 women, 4 men, aged 27-42 years) after oral intake of 300 mg adenosine (Sigma Aldrich Chemie GmbH, Taufkirchen, Germany) every 24 hours over 7 days.

Adverse events of adenosine intake were not reported.

The participant selection and laboratory analysis was carried out as described in example 1 referring to the study on dipyridamole. EPCs were measured on days 0, 3, and 7 at 0 h and 6 h after treatment and 7 days after discontinuation of the treatment (post-dose).

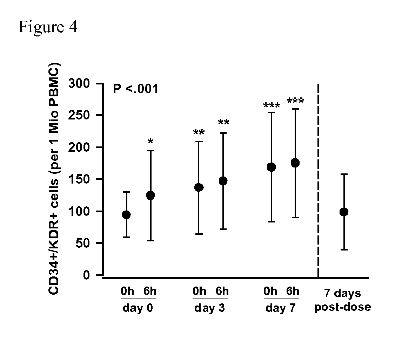

As can be seen from Figure 4 the amount of endothelial progenitor cells increased after the oral intake of adenosine. After discontinuing the medication the amount of endothelial progenitor cells decreased to the pre-dose level.

These results support the hypothesis that adenosine itself is the causal agent regulating the number of circulating EPC. Administration of 300 mg adenosine per os once a day was sufficient to cause a clinically significant increase of EPC numbers without any side effects.

In summary, these results show that administration of compounds which increase the plasma levels of adenosine elevate the levels of circulating endothelial progenitor cells (EPC). Since reduced numbers of EPC were found to be a strong risk factor of mortality and major cardiovascular events in chronic ischemic conditions such as coronary heart disease, pharmacological elevation of EPCs may represent a causal, cost efficient, low-risk treatment opportunity for primary and secondary prevention of ischemic cardiovascular events.

## Claims

1. Use of adenosine level affecting-compounds selected from the group comprising dipyridamole, mopidamol, dilazep, draflazine, 2,6-Bis(diethanolamino)-4,8-diheptamethyleneimino-pyrimido[5,4-*d*]pyrimidine, RA-642, adenosine, adenosine analogues, adenosine A_{2(A)} receptor agonists, inhibitors of adenosine kinase, activators of ecto-5'-nucleotidase and/or pharmaceutically acceptable salts thereof for the manufacture of a medicament for the therapeutic or prophylactic treatment and/or diagnosis of clinical conditions resulting from a deficit of endothelial progenitor cells.

2. Use of adenosine level affecting-compounds according to claim 1, **characterized in that** the clinical condition resulting from a deficit of endothelial progenitor cells is a disease selected from the group comprising coronary heart disease and/or vascular diseases preferably selected from the group comprising atherosclerosis, diabetes mellitus, hypertension, metabolic syndrome, ischemic stroke, peripheral arterial disease, valvular heart disease, Raynaud syndrome, erectile dysfunction, chronic obstructive pulmonary disease, rheumatoid arthritis and other autoimmune disorders with vascular involvement, human immunodeficiency virus infection and other chronic infective disorders with vascular involvement, retinal vascular defects, graft vasculopathy, renal insufficiency and/or hemodialysis.

3. Use of adenosine level affecting-compounds according to claims 1 or 2, **characterized in that** dipyridamole is formulated as a sustained release formulation, wherein the sustained release formulation preferably is an oral formulation selected from the group comprising sustained release capsules and/or sustained release tablets.

4. Use of adenosine level affecting-compounds according to any one of the aforementioned claims, **characterized in that** dipyridamole is used in combination with a compound selected from the group comprising acetylsalicylic acid, statins, ACE inhibitors, PPARgamma agonists, glucocorticoids, clopidogrel, ticlopidin, prasugrel, ticagrelor, cangrelor, PRT 060128 and/or SCH 530348.

5. Use of adenosine level affecting-compounds according to any one of the aforementioned claims, **characterized in that** dipyridamole is administered in a daily dosage in the range of ≥ 25 mg to ≤ 500 mg, preferably in the range of ≥ 100 mg to ≤ 400 mg, more preferably in the range of ≥ 300 mg to ≤ 400 mg.

6. Use of adenosine level affecting-compounds according to any one of the aforementioned claims, **characterized in that** the adenosine analogues are selected from the group comprising 5'-N-Ethylcarboxamidoadenosine, L-phenylisopropyl-adenosine, 2-chloroadenosine and/or N6-cyclopentyladenosine.

7. Use of adenosine level affecting-compounds according to any one of the aforementioned claims, **characterized in that** the adenosine A_{2(A)} agonists are selected from the group comprising 2-p-(2-Carboxyethyl)phenethylamino-5'-N-ethylcarboxamidoadenosine hydrochloride hydrate.

8. Adenosine level affecting-compounds selected from the group comprising dipyridamole, mopidamol, dilazep, draflazine, 2,6-Bis(diethanolamino)-4,8-diheptamethyleneimino-pyrimido[5,4-*d*]pyrimidine, RA-642, adenosine, adenosine analogues, adenosine A_{2(A)} receptor agonists, inhibitors of adenosine kinase, activators of ecto-5'-nucleotidase and/or pharmaceutically acceptable salts thereof for use in the therapeutic or prophylactic treatment and/or diagnosis of clinical conditions resulting from a deficit of endothelial progenitor cells.

9. A pharmaceutical or diagnostic composition for the therapeutic and/or prophylactic treatment and/or the diagnosis of clinical conditions resulting from a deficit of endothelial progenitor cells comprising an adenosine level affecting-compound selected from the group comprising dipyridamole, mopidamol, dilazep, draflazine, 2,6-Bis(diethanolamino)-4,8-diheptamethyleneimino-pyrimido[5,4-d]pyrimidine, RA-642, adenosine, adenosine analogues, adenosine A_{2(A)} receptor agonists, inhibitors of adenosine kinase, activators of ecto-5'-nucleotidase and/or pharmaceutically acceptable salts thereof.

10. A method of detecting and/or diagnosing a clinical condition resulting from a deficit of endothelial progenitor cells in a subject, the method comprising the steps of:
- providing a biological sample from a patient;
- detecting and/or quantifying the level of adenosine in the plasma and/or erythrocytes of the biological sample;
- detecting and/or quantifying the level of adenosine-depending activity markers;
- determining the standardised number of endothelial progenitor cells;
- determining the correlation of adenosine and adenosine-depending activity markers with the standardised number of endothelial progenitor cells; and
- comparing the level of adenosine in the plasma and/or erythrocytes of the biological sample with that in a control sample,
wherein a different expression level of adenosine and/or adenosine-depending activity markers in the biological sample compared to that in the control sample indicates the presence of a clinical condition resulting from a deficit of endothelial progenitor cells in the patient.

11. A method for treating a clinical condition resulting from a deficit of endothelial progenitor cells in a subject, the method comprising administering to the subject a therapeutically effective amount of an adenosine level affecting-compounds selected from the group comprising dipyridamole, mopidamol, dilazep, draflazine, 2,6-Bis(diethanolamino)-4,8-diheptamethyleneimino-pyrimido[5,4-*d*]pyrimidine, RA-642, adenosine, adenosine analogues, adenosine A_{2(A)} receptor agonists, inhibitors of adenosine kinase, activators of ecto-5'-nucleotidase and/or pharmaceutically acceptable salts thereof.

12. The method according to claim 11, **characterized in that** the clinical condition resulting from a deficit of endothelial progenitor cells is a disease selected from the group comprising coronary heart disease and/or vascular diseases preferably selected from the group comprising atherosclerosis, diabetes mellitus, hypertension, metabolic syndrome, ischemic stroke, peripheral arterial disease, valvular heart disease, Raynaud syndrome, erectile dysfunction, chronic obstructive pulmonary disease, rheumatoid arthritis and other autoimmune disorders with vascular involvement, human immunodeficiency virus infection and other chronic infective disorders with vascular involvement, retinal vascular defects, graft vasculopathy, renal insufficiency and/or hemodialysis.

13. The method according to claims 11 or 12, **characterized in that** dipyridamole is formulated as a sustained release formulation, wherein the sustained release formulation preferably is an oral formulation selected from the group comprising sustained release capsules and/or sustained release tablets.

14. The method according to any one of the aforementioned claims, **characterized in that** dipyridamole is used in combination with a compound selected from the group comprising acetylsalicylic acid, statins, ACE inhibitors, PPARgamma agonists, glucocorticoids, clopidogrel, ticlopidin, prasugrel, ticagrelor, cangrelor, PRT 060128 and/or SCH 530348.
